# FASCICULE DE BREVET EUROPEEN

(11) **EP 0 703 732 B1**
(45) Date de publication et mention de la délivrance du brevet: **17.09.1997**
(21) Numéro de dépôt: 94919732.1
(22) Date de dépôt: 17.06.1994
(51) Int. Cl.: A23L 1/305, A61K 31/195

(54) **COMPOSITION A USAGE ALIMENTAIRE ET/OU PHARMACEUTIQUE PAUVRE EN POLYAMINES**
POLYAMINE NÄHR-UND/ODER HEILMITTELZUSAMMENSETZUNG
FOOD AND/OR PHARMACEUTICAL COMPOSITION HAVING A LOW POLYAMINE CONTENT

(30) Priorité: 17.06.1993 FR 9307586
(43) Date de publication de la demande: 03.04.1996
(73) Titulaire: UNIVERSITE DE RENNES I, F-35000 Rennes (FR)
(72) Inventeur: MOULINOUX, Jacques-Philippe, F-35000 Rennes (FR); QUEMENER, Véronique, F-35700 Rennes (FR); JAUSSAN, Véronique, F-14000 Caen (FR)
(74) Mandataire: Vidon, Patrice
(86) Numéro de dépôt international: FR9400737
(87) Numéro de publication internationale: WO9500042

(56) Documents cités:
- EP-A- 0 162 413
- EP-A- 0 248 217
- US-A- 4 112 123
- US-A- 4 201 788
- US-A- 4 207 315
- US-A- 4 559 362
- US-A- 5 162 373
- CELLULAR AND MOLECULAR BIOLOGY, vol.37, no.8, 1991, UK pages 773 - 783 J.P.MOULINOUX ET AL. 'Biological significance of circulating polyamines in oncology' cité dans la demande
- ANTICANCER RESEARCH, vol.11, no.1, Février 1991 pages 175 - 180 J.P.MOULINOUX ET AL. 'Inhibition of the growth of U-251 human glioblastoma in nude mice by polyamine deprivation' cité dans la demande
- ANTICANCER RESEARCH, vol.12, 1992 pages 1447 - 1454 V.QUEMENER ET AL. 'Polyamine dep'rivation enhances antitumoral efficacy of chemotherapy' cité dans la demande
- CANCER RESAERCH, vol.50, Août 1990 pages 5077 - 5083 N.SEILER ET AL. 'Endogenous and exogenous polyamines in support of tumor growth' cité dans la demande

## Description

L'invention concerne à la fois le domaine alimentaire et le domaine pharmaceutique.

Les polyamines et tout particulièrement la putrescine (I), la spermidine (II) et la spermine (III) sont présentes dans toutes les cellules.

⁺NH₃ - (CH₂)₄ - NH₃⁺ (I)

⁺NH₃ - (CH₂)₃ - NH - (CH₂)₄ - NH₃⁺ (II)

⁺NH₃ - (CH₂)₃ - NH - (CH₂)₄ - NH - (CH₂)₃ - NH₃⁺ (III)

Bien que ces molécules aient été longtemps considérées comme dénuées de tout rôle physiologique et ne représentant qu'une étape terminale du catabolisme tissulaire, de nombreux travaux scientifiques ont montré que les polyamines issues de la décarboxylation de l'ornithine étaient en fait des molécules biologiquement actives et capables d'intervenir à différents niveaux importants de la vie de la cellule.

Ces molécules que l'on trouve non seulement à l'intérieur même des cellules mais également à l'état circulant dans les liquides biologiques de l'organisme, tels que le sang sont issues de trois sources principales :
- la prolifération cellulaire physiologique (croissance et/ou renouvellement des cellules constitutives de l'organisme) et tumorale,
- l'alimentation,
- les bactéries intestinales.

Les polyamines intracellulaires ne proviennent pas simplement d'échanges transmembranaires passifs avec les liquides biologiques de l'organisme mais résultent d'un métabolisme finement régulé et en relation avec le cycle cellulaire. Un accroissement de l'activité de l'enzyme responsable de la voie anabolique intracellulaire principale des polyamines, l'ornithine-décarboxylase (ODC), et donc des concentrations en putrescine, spermidine et spermine est en effet observé en fin de phase G1 et début de phase S, et en début de phase G2 du cycle de réplication cellulaire. L'implication du métabolisme des polyamines dans la réplication cellulaire et donc dans les processus prolifératifs font de ce métabolisme l'une des cibles privilégiées des drogues antiprolifératives, en même temps que la source de nouveaux signaux circulants susceptibles de révéler l'existence d'un processus néoplasique au sein d'un organisme.

Il a été constaté que la croissance tumorale s'accompagne en règle générale d'un accroissement très significatif de l'anabolisme intracellulaire des polyamines qui se retrouvent alors en quantité augmentée dans les liquides biologiques. Bien que le rôle exact des polyamines circulantes en tant que paramètres impliqués dans la régulation (ou la dysrégulation) homéostatique de la prolifération cellulaire ne soit pas élucidé, il a été prouvé que les taux érythrocytaires de polyamines pouvaient être considérés et utilisés comme des marqueurs du niveau hyperplasique tumoral (voir "Biological significance of circulating polyamines in oncology" - Moulinoux, J.-Ph, Quemener, V. et Khan, N.A. - *Cellular and molecular biology* -37(8), 773-783, 1991). Ces travaux ont par ailleurs montré que les polyamines circulantes, provenant soit des cellules elles-mêmes soit du tractus gastro-intestinal (alimentation, bactéries intestinales) participaient au maintien de l'état prolifératif malin.

On connaît dans l'état de la technique divers inhibiteurs de l'ornithinedécarboxylase et notamment des inhibiteurs sélectifs irréversibles de cette enzyme tels que l'α-difluorométhylornithine (α-DFMO) commercialisée par la société Marion Merrell Dow sous la dénomination Eflornithine*™*. Toutefois, l'administration isolée d'α-DFMO en l'absence de tout autre traitement à des patients ou à des animaux cancéreux, s'est révélée inefficace vis-à-vis de la progression tumorale maligne, ceci alors même que cet inhibiteur de la synthèse des polyamines, tout en diminuant de manière très significative les taux intracellulaires de putrescine et de spermidine, inhibait totalement la prolifération cellulaire cancéreuse *in vitro*. L'incapacité de l'α-DFMO à réduire *in vivo* la prolifération cellulaire maligne est très probablement en relation avec le fait que, dans l'organisme, les cellules cancéreuses restaurent la quantité intracellulaire de polyamines nécessaire au maintien de leur prolifération en captant les polyamines présentes dans le milieu extracellulaire, c'est-à-dire dans les différents liquides biologiques dont le sang. Cette hypothèse a été confortée par les travaux de Person et al (*Cancer Research,* 1988, n°48, pp. 4807 à 4811) qui ont montré qu'à la différence de ce que l'on constatait avec des cellules sauvages L1210, des souris inoculées avec des cellules L1210 mutées, rendues incapables de capter les polyamines extracellulaires, répondaient favorablement à un traitement inhibiteur de la biosynthèse de polyamines intracellulaires par l'α-DFMO.

Différents travaux ont par ailleurs mis en évidence que, chez l'animal, l'administration conjointe :
- d'une alimentation dépourvue de polyamines,
- d'α-DFMO,
- d'un inhibiteur de la polyamine-oxydase (PAO) supprimant la rétroconversion oxydative de la spermidine et de la spermine en putrescine, et
- de néomycine et de métronidazole,
entraîne une inhibition quasi-totale de la progression tumorale du carcinome pulmonaire de Lewis 3LL (Seiler N. et al, *Cancer Research*, 1990, n°50, pp. 5077-5083), du glioblastome humain U251 (Moulinoux J-Ph. et al, *Anticancer Research,* 1991, n°11, pp. 175-180), de l'adénocarcinome de la prostate Dunning MAT-LyLu (Moulinoux J-Ph. et al, *Journal of Urology*, 1991, n° 146, pp. 1408,1412) et du neuroblastome humain neuro 2a (Quemener et al, "Polyamines in the gastro-intestinal tract", Dowling R.H., Fölsch I.R. et Löser C Ed., *Kluwer Academic Publishers Boston*, 1992 pp. 375-385).

Il a par ailleurs été également démontré chez l'animal que la déplétion en polyamines pouvait considérablement potentialiser les effets antiprolifératifs des drogues antitumorales conventionnelles (méthotrexate, cyclophosphamide, vindesine) tout en allongeant le temps de survie des animaux et pouvait permettre de réduire les quantités de drogues administrées tout en conservant le même effet antitumoral (Quemener V. et al, "Polyamine deprivation enhances antitumoral efficacy of chemotherapy", *Anticancer Research* n°12, 1992, pp. 1447-1454).

Le document US-A-4 112 123 décrit une composition alimentaire pour patients qui n'ont pas un état catabolique normal.

L'objectif de l'invention est de fournir une composition administrable à l'homme à titre d' aliment, de complément alimentaire et/ou de composition pharmaceutique, permettant de pallier l'inefficacité des traitements réalisés jusqu'ici chez l'homme par administration isolée d'α-DFMO.

Plus précisément, l'objectif a été de mettre au point une composition efficace chez l'homme et, en particulier, une formulation précise des constituants de la composition inventive permettant une réelle synergie des effets provoqués, chez l'homme, par la diminution des apports extérieurs de polyamines et par l'inhibition de la synthèse intracellulaire de ces composés.

Cet objectif est atteint grâce à une composition pouvant être ingérée par l'homme, caractérisée en ce qu'elle est constituée d'un mélange nutritif pauvre en polyamines contenant moins de 50 picomoles/g de putrescine, de spermidine, de spermine et cadavérine, comprenant, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes.

Cette composition pourra être présentée sous forme sèche à dissoudre extemporanément dans un véhicule neutre convenant a l'administration orale ou encore entérale, ou sous forme liquide prête à l'emploi. Dans tous les cas, la composition est présentée sous forme stérile.

Une telle composition est particulièrement bien adaptée à l'homme et constitue un substitutif alimentaire qui permet de carencer les patients de façon efficace en polyamines. Une telle composition permet en effet d'alimenter un patient de façon satisfaisante tout en induisant une carence en polyamines, d'une part en inhibant la synthèse intracellulaire de polyamines et d'autre part en diminuant l'apport de polyamines exogènes. Cette composition apporte, journellement, au moins 60 fois moins de putrescine, 200 fois moins de cadavérine, 700 fois moins de spermidine et de spermine que l'alimentation naturelle humaine la plus pauvre qui soit en teneur de polyamines, mais qui réponde néanmoins aux besoins nutritionnels humains.

Une telle composition permet d'inhiber fortement la synthèse endogène des polyamines et diminue de façon très importante l'apport en ces composés puisque les différents ingrédients qui la constituent en sont quasiment dépourvus. Afin de diminuer également les apports en polyamines par les bactéries intestinales, cette composition pourra être administrée concomitamment à une décontamination du tractus gastro-intestinal au moyens d'antibiotique(s) et/ou d'antiparasitaire(s), tels que, par exemple, la néomycine et le métronidazole. On pourra d'ailleurs envisager d'inclure de tels antibiotique(s) et/ou antiparasitaire(s) directement dans ladite composition, sans sortir du cadre de l'invention.

Les nutriments utilisés dans la composition alimentaire selon l'invention possèdent une bonne valeur nutritionelle même chez les sujets malades.

La quantité d'eau employée pour réaliser la composition est déterminée pour que la composition soit plus ou moins liquide et puisse être facilement ingérée par le patient.

Le pourcentage pondéral du mélange constitué de vitamines, de minéraux et d'électrolytes est choisi de façon à répondre aux proportions, connues de l'homme du métier, devant se trouver dans une alimentation équilibrée.

Une telle composition pourra être administrée conjointement à au moins un inhibiteur de la synthèse intracellulaire des polyamines.

Selon une variante intéressante de l'invention ladite composition est enrichie avec au moins un inhibiteur de la synthèse intracellulaire des polyamines à raison d'au plus 15% en poids par rapport au poids sec total de la composition et, préférentiellement, à raison d'une quantité comprise entre 0,2% et 3% en poids.

Les inhibiteurs de l'ODC utilisables sont choisis en particulier parmi les composés suivants :

### Antagonistes du phosphate de pyridoxal

. L-canaline
. N-(5'-phosphopyridoxyl)ornithine

### Inhibiteurs compétitifs

. α-hydrazino-ornithine
. acide DL-α-hydrazino-δ-aminovalérique
. α-méthylornithine (α-MO)
. *trans-3-déhydro-DL-ornithine*
. 1,4-diamino-*trans*-2-butène
. 1,4-diaminobutanone

### Inhibiteurs diaminés

. 1,3-diaminopropane
. 1,3-diamino-2-propanol
. bis(éthyl)spermine

### Inhibiteurs suicides et irréversibles

. 2-difluorométhylornithine (DFMO)
. monofluorométhylornithine
. 2-monofluorométhyldéhydro-ornithine
. 2-monofluorométhyldéhydro-ornithine méthyl ester
. 5-hexyne-1,4-diamine
. *trans*-hex-2-èn-5-yne-1,4-diamine
. monofluorométhylputrescine
. difluorométhylputrescine
. α-allénylputrescine
. (2R,5R)-6-heptyne-2,5-diamine.

Parmi ces inhibiteurs, les inhibiteurs compétitifs sont particulièrement préférés et notamment l'α-méthylornithine (α-MO).

L'α-méthylornithine montre de nombreux avantages dans le cadre de l'utilisation proposée ici. En effet, l'α-MO présente l'intérêt d'être un composé naturel facilement synthétisable et a une constante d'inhibition élevée.

L'α-méthylornithine présente de plus l'avantage d'inhiber la synthèse des polyamines chez Escherischia coli, la bactérie la plus commune peuplant naturellement le tractus intestinal, ce qui n'est notamment pas le cas de l'α-DFMO.

Ainsi l'emploi d'une composition alimentaire selon l'invention contenant en tant qu'inhibiteur de la synthèse intracellulaire des polyamines, de l'α-méthylornithine est-elle susceptible de réduire l'apport exogène de polyamines par les bactéries intestinales sans pour autant avoir recours à une antibiothérapie concomitamment à l'administration de cette composition ou, pour le moins, en permettant de diminuer la dose d'antibiotiques administrée.

Enfin, l'α-MO présente l'avantage d'être un simple inhibiteur compétitif de l'ornithine décarboxylase (ce qui va à l'encontre des pistes actuellement explorées par la communauté scientifique internationale) et diminue fortement les risques d'accoutumance de l'organisme par mutation aboutissant à une résistance cellulaire accrue.

La conception originale de l'invention privilégie la recherche d'un effet de synergie entre plusieurs facteurs, à savoir notamment l'inhibition de la synthèse intracellulaire des polyamines et l'apport carencé conjoint en ces composés. Sous cet angle, il apparaît également que le meilleur inhibiteur est l'α-MO.

Selon une variante, la composition selon l'invention est enrichie en vitamines, notamment celles apportées, chez l'être humain sain, par les bactéries intestinales. En effet, l'antibiothérapie qui peut accompagner l'administration de ladite composition peut aussi conduire à diminuer l'apport en certaines vitamines. Dans ce cas, il peut s'avérer nécessaire d'enrichir la composition selon l'invention en ces vitamines afin de ne pas provoquer de carence vitaminique à la suite de l'administration prolongée de ladite composition. Notamment, il pourra s'avérer utile d'enrichir la composition en vitamines ou en dérivés de vitamines. Certains dérivés de la vitamine A (acide rétinoïque) sont en effet des inhibiteurs de l'activité ODC.

Préférentiellement, les glucides de la composition appartiennent au groupe comprenant les polymères de glucose, les maltodextrines, le saccharose, les amidons modifiés, le glucose monohydraté, le sirop de glucose déshydraté, le monostéarate de glycérol et leurs mélanges. De tels glucides sont en effet digestibles même en cas de pathologie digestive ou d'anomalies fonctionnelles secondaires à un traitement anticancéreux (chimiothérapie ou radiothérapie).

Selon une variante de l'invention, les protéines utilisées appartiennent au groupe comprenant les protéines solubles du lait, les protéines de soja, les peptides de sérum, le jaune d'oeuf en poudre, le caséinate de potassium, les peptides non phosphorylés, les peptides de caséine, le caséinate mixte, l'isolat de soja et leurs mélanges.

Préférentiellement, les lipides appartiennent au groupe comprenant l'huile de beurre, l'huile d'arachide, les triglycérides à chaîne moyenne, l'huile de pépins de raisin, l'huile de soja, l'huile d'onagre et leurs mélanges. Avantageusement, lesdits lipides sont constitués par un mélange d'au moins une huile d'origine animale, d'au moins une huile d'origine végétale et de stéarate de glycérol.

Selon une variante de l'invention ladite composition constitue une ration journalière alimentaire d'un être humain et comprend :
- éventuellement ledit inhibiteur de la synthèse intracellulaire des polyamines à raison de moins de 50g et préférentiellement à raison de 1 à 10 g,
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

Les quantités de vitamines, de minéraux et d'électrolytes utilisés sont connues de l'homme du métier et peuvent être facilement trouvées dans la littérature (voir par exemple,"Apports nutritionnels conseillés" Dupin, Abraham et Giachetti deuxième édition 1992, Ed. TEC et DOC Lavoisier)

Une telle composition permet, à elle seule, de répondre aux besoins nutritionnels journaliers d'un patient tout en permettant de réduire la synthèse intracellulaire et l'apport extérieur en polyamines. Elle constitue alors un aliment à part entière.

Bien sûr, il pourra être envisagé d'administrer une telle composition non pas en une seule prise mais en plusieurs prises espacées au cours de la même journée. Chaque ration sera alors définie pondéralement de façon à constituer un sous-multiple d'une ration journalière alimentaire d'un être humain et comprendra:
- éventuellement ledit inhibiteur de la synthèse intracellulaire des polyamines à raison de moins de 50/X g et préférentiellement à raison de 1/X à 10/X g,
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain.
X étant un entier compris entre 2 et 8 et correspondant au nombre de rations devant être ingérées par le patient pour satisfaire ses besoins nutritionnels journaliers.

Le nombre de telles rations pourra être choisi de façon à répondre totalement aux besoins alimentaires journaliers du patient ou bien être choisi de façon à ne couvrir qu'une partie des besoins nutritionnels de celui-ci, le reste de ces besoins étant assurés par un alimentation naturelle pauvre en polyamines (jambon et pâtes ou riz par exemple). Dans ce cas la composition alimentaire sera utilisée en tant que complément alimentaire.

L'invention a également pour objet un agent à deux composants A et B, le composant A étant constitué par une composition pouvant être ingérée par l'homme constituée d'un mélange nutritif pauvre en polyamines contenant moins de 50 picomoles/g de putrescine, de spermidine, de spermine et de cadavérine et comprenant, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes, et un composé B, constitué par un inhibiteur de synthèse intracellulaire des polyamines, constitué de préférence par l'α-méthylornithine, les composants A et B étant mis en oeuvre comme produits de combinaison pour une utilisation simultanée, séparée ou décalée dans le temps pour le traitement du cancer, en particulier du cancer de la prostate.

La composition ou l'agent selon l'invention pourront être utilisés à titre de médicament, d'aliment, de supplément alimentaire ou encore de produit de nutrition thérapeutique.

La composition ou l'agent selon l'invention sont susceptibles de présenter une activité anti-néoplasique chez l'homme. La déplétion en polyamines potentialisant les effets des traitements anticancéreux conventionnels, la composition ou l'agent selon l'invention seront utilisables en association avec d'autres traitements anticancéreux, y compris ceux faisant appel à des inhibiteurs du métabolisme des polyamines.

En particulier, une telle composition ou un tel agent sont susceptibles de se révéler efficaces pour le traitement du cancer de la prostate.

L'invention sera plus facilement comprise grâce aux deux exemples de réalisation de l'invention qui vont suivre.

### Exemple 1

On prépare la composition suivante (voir tableau IV).
On disperse dans 150 litres d'eau,
- 786 g de phosphate disodique,
- 5,080 kg d'huile d'arachide,
- 0,300 kg de stéarate de glycérol,
- 1,280 kg d'huile de beurre,
après agitation, on y incorpore :
- 8,760 kg de concentré de protéine soluble de lait, et ensuite 18,750 kg de maltodextrine, 7,000 kg de saccharose.

L'ensemble est refroidi à température ambiante et on ajoute 45 g d'oxyde de magnésium, 13,8 g de carbonate de calcium, 227,5 g de chlorure de magnésium, 6,7 g de sulfate de fer, 40 g de prémix vitaminique et de sels minéraux, 73,6 g de chlorure de choline, 60,0 g d'acide ascorbique.

On ajuste ensuite le volume de la cuve à 200 litres, par addition d'eau, et le pH est ajusté à 7,10 par addition d'une solution d'hydroxyde de sodium 2N.

Après dégazage, stérilisation, homogénéisation, on introduit le produit dans des boîtes métalliques serties. On ajoute à cette composition, lorsqu'on souhaite une composition complète, 4,0kg d'α-méthylornithine.

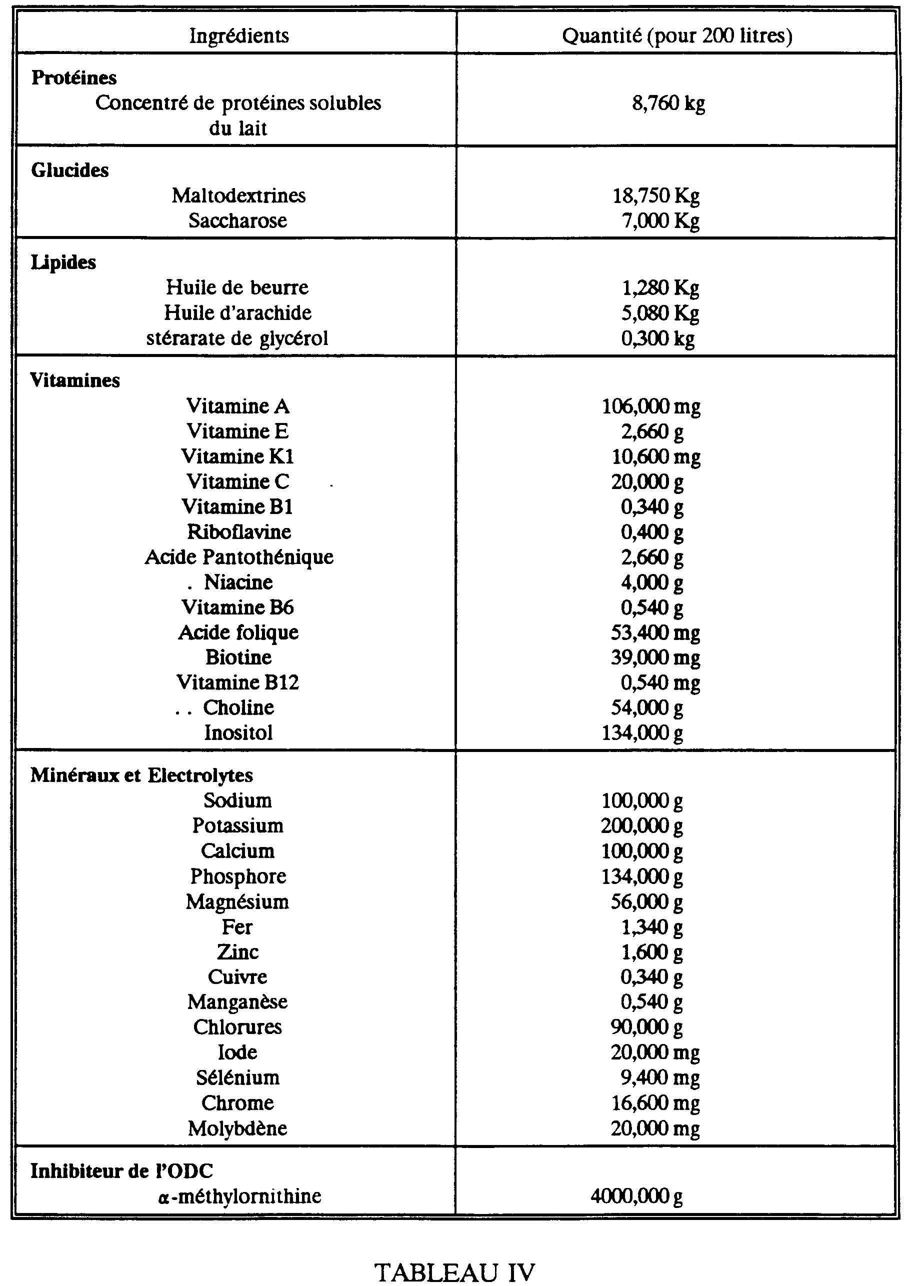

La composition centésimale obtenue est la suivante :
Glucides : 12,500 g dont 9,000 g de maltodextrines et 3,500 g de saccharose ;
Lipides : 3,330g dont 0,640 g d'huile de beurre, 2,540 g d'huile d'arachide et 0,150 g de stéarate de glycérol ;
Protéines : 4,000 g apportées par un concentré de protéines solubles de lait.
Vitamines, minéraux et électrolytes : 0,600 g ;
α-méthylornithine : 3,000 g ;
eau : QSP 100 ml.

Le pourcentage pondéral de vitamines, de minéraux et d'électrolytes utilisé est choisi de façon telle que la consommation journalière de quatre boîtes permette de répondre aux besoins nutritionnels conseillés par Dupin, Abraham et Giachetti ("Apports nutritionnels conseillés pour la population française" deuxième édition 1992, Editions TEC et DOC Lavoisier).

### Exemple 2

Une préparation présentant sensiblement les mêmes ingrédients que ceux indiqués dans le tableau IV est préparée selon la même procédé que celui détaillé dans l'exemple 1. Toutefois, la source de protéines est remplacée par un mélange d'isolat de soja et de poudre de jaune d'oeufs. L'huile d'arachide est quant à elle substituée par un mélange d'huile de germes de maïs et d'huile de soja.

La composition centésimale obtenue par boîte de 100 ml est la suivante :
Glucides : 12,500 g dont 9,000 g de maltodextrines et 3,500 g de saccharose ;
Lipides : 3,330g dont 0,640 g d'huile de beurre, 0,840g d'huile de germes de maïs, 1,700 g d'huile de soja et 0,150 g ;
Protéines : 4,000 g dont 3,380 g d'isolat de soja et 0,620 g de poudre de jaune d'oeufs ;
Vitamines, minéraux et électrolytes : 0,600g
α-méthylornithine : 3,000 g
eau : QSP 100 ml.

Les deux exemples de réalisation ici décrits n'ont pas pour objet de réduire la portée de l'invention. En particulier, il pourra être envisagé d'employer un autre inhibiteur de la synthèse intra-cellulaire des polyamines que l'α-méthylornithine et d'incorporer cet inhibiteur dans des proportions pondérales plus importantes que celle mentionnée dans ces exemples. Il pourra également être envisagé d'utiliser d'autres sources de glucides, de lipides ou de protéines que celles mentionnées sans sortir du cadre de l'invention.

## Revendications

1. Composition pouvant être ingérée par l'homme caractérisée en ce qu'elle est constituée d'un mélange nutritif pauvre en polyamines contenant moins de 50 picomoles/g de putrescine, de spermidine, de spermine et de cadavérine, comprenant, en pourcentage de poids sec par rapport au poids sec total : 10 % à 35 % de lipides, 8 % à 30 % de protéines, 35 % à 80 % de glucides, jusqu'à 10 % d'un mélange constitué de vitamines, de minéraux et d'électrolytes.

2. Composition selon la revendication 1 caractérisée en ce qu'elle est enrichie avec au moins un inhibiteur de la synthèse intracellulaire des polyamines à raison d'au plus 15 % par rapport au poids sec total de la composition.

3. Composition selon la revendication 2 caractérisée en ce qu'elle est enrichie avec ledit inhibiteur à raison de 0,2 % à 3 % en poids par rapport au poids sec total de la composition.

4. Composition selon la revendication 2 ou 3 caractérisée en ce que ledit inhibiteur est un inhibiteur compétitif de l'ornithine décarboxylase.

5. Composition selon la revendication 4 caractérisée en ce que ledit inhibiteur compétitif est l'α-méthylornithine.

6. Composition selon l'une des revendications 1 à 5 caractérisée en ce qu'elle est enrichie en vitamines.

7. Composition selon l'une des revendications 1 à 6 caractérisée en ce que lesdits glucides appartiennent au groupe comprenant les polymères de glucose, les maltodextrines, le saccharose, les amidons modifiés, le glucose monohydraté, le sirop de glucose déshydraté, le monostéarate de glycérol et leurs mélanges.

8. Composition selon l'une des revendications 1 à 7 caractérisée en ce que lesdites protéines appartiennent au groupe comprenant les protéines solubles du lait, les protéines de soja, les peptides de sérum, le jaune d'oeuf en poudre, le caséinate de potassium, les peptides non phosphorylés, les peptides de caséine, le caséinate mixte, l'isolat de soja et leurs mélanges.

9. Composition selon l'une quelconque des revendications 1 à 8 caractérisée en ce que lesdits lipides appartiennent au groupe comprenant l'huile de beurre, l'huile d'arachide, les triglycérides à chaîne moyenne, l'huile de pépins de raisin, l'huile de soja, l'huile d'onagre et leurs mélanges.

10. Composition selon l'une des revendications 1 à 9 caractérisée en ce que lesdits lipides sont constitués par un mélange d'au moins une huile d'origine animale, d'au moins une huile d'origine végétale et de stéarate de glycérol.

11. Composition selon l'une des revendications 1 et 6 à 10 caractérisée en ce qu'elle constitue la ration journalière alimentaire d'un être humain et en ce qu'elle comprend :
- entre 75 g et 500 g de glucides
- entre 20 g et 185 g de lipides
- entre 20 g et 225 g de protéines
- des vitamines, des minéraux et des électrolytes en quantités suffisantes pour répondre aux besoins nutritionnels journaliers d'un être humain.

12. Composition selon l'une des revendications 1 à 10 caractérisée en ce qu'elle constitue la ration journalière alimentaire d'un être humain et en ce qu'elle comprend :
- moins de 50 g et, préférentiellement, entre 1 à 10 g dudit inhibiteur de la synthèse intracellulaire des polyamines,
- entre 75 g et 500 g de glucides,
- entre 20 g et 185 g de lipides,
- entre 20 g et 225 g de protéines,
- des vitamines, des minéraux et des électrolytes en quantités suffisantes pour répondre aux besoins nutritionnels journaliers d'un être humain.

13. Composition selon l'une des revendications 1 et 6 à 10 caractérisée en ce qu'elle est un sous-multiple d'une ration journalière alimentaire d'un être humain et en ce qu'elle comprend :
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain,
et
X étant en outre compris entre 2 et 8 et correspondant au nombre de rations devant être ingérées par le patient pour satisfaire ses besoins nutritionnels journaliers.

14. Composition selon l'une des revendications 1 à 10 caractérisée en ce qu'elle est un sous-multiple d'une ration journalière alimentaire d'un être humain et en ce qu'elle comprend :
- moins de 50/X g et, préférentiellement, entre 1/X et 10/X g dudit inhibiteur de la synthèse intracellulaire des polyamines,
- entre 75/X g et 500/X g de glucides,
- entre 20/X g et 185/X g de lipides,
- entre 20/X g et 225/X g de protéines,
- des vitamines, des minéraux et des électrolytes en quantités suffisantes pour répondre partiellement aux besoins nutritionnels journaliers d'un être humain,
et
X étant un entier compris entre 2 et 8 et correspondant au nombre de rations devant être ingérées par le patient pour satisfaire ses besoins nutritionnels journaliers.

15. Composition selon l'une des revendications 1 à 14 caractérisée en ce qu'elle se présente sous une forme sèche à dissoudre extemporanément dans un véhicule neutre.

16. Composition selon l'une des revendications 1 à 14 caractérisée en ce qu'eue inclut un véhicule neutre la rendant prête à l'emploi.

17. Agent à deux composants A et B, le composant A étant constitué par une composition pouvant être ingérée par l'homme selon l'une des revendications 1, 6 à 11, 13, 15 ou 16 et le composé B étant constitué par un inhibiteur de synthèse intracellulaire des polyamines, les composants A et B étant mis en oeuvre comme produits de combinaison pour une utilisation simultanée, séparée ou décalée dans le temps.

18. Agent selon la revendication 17 caractérisé en ce que ledit inhibiteur est de l'α-méthylornithine.

19. Composition ou agent selon l'une quelconque des revendications 1 à 18 pour une utilisation appartenant au groupe comprenant les aliments, suppléments alimentaires et produits de nutrition.

20. Composition ou agent selon l'une quelconque des revendications 1 à 18 utilisable comme médicament.

21. Composition ou agent selon l'une des revendications 19 ou 20 utilisable comme produit de nutrition thérapeutique.

22. Composition ou agent selon l'une quelconque des revendications 19 à 21 utilisable comme agent anti-cancéreux.

23. Composition ou agent selon la revendication 22 utilisable pour le traitement du cancer de la prostate.

## Claims

1. Composition that can be ingested by man, characterized in that it consists of a nutritive mixture low in polyamines and containing less than 50 picomoles/g of putrescine, spermidine, spermine and cadaverine, comprising, as a percentage in dry weight with respect to the total dry weight : 10 % to 35 % of lipids, 8 % to 30 % of proteins, 35 % to 80 % of glucides and up to 10 % of a mixture consisting of vitamins, minerals and electrolytes.

2. Composition according to claim 1, characterized in that it is enriched with at least one inhibitor of the intracellular synthesis of polyamines in a quantity of up to 15 % with respect to the total dry weight of the composition.

3. Composition according to claim 2, characterized in that it is enriched with the said inhibitor in a quantity of 0.2 % to 3 % by weight with respect to the total dry weight of the composition.

4. Composition according to claim 2 or 3, characterized in that the said inhibitor is a competitive inhibitor of ornithine decarboxylase.

5. Composition according to claim 4, characterized in that the said competitive inhibitor is α-methylornithine.

6. Composition according to one of claims 1 to 5, characterized in that it is enriched with vitamins.

7. Composition according to one of claims 1 to 6, characterized in that the said glucides belong to the group comprising glucose polymers, maltodextrins, saccharose, modified starches, monohydrated glucose, dehydrated glucose syrup, glycerol monostearate and mixtures thereof.

8. Composition according to one of claims 1 to 7, characterized in that the said proteins belong to the group comprising the soluble proteins of milk, soya proteins, serum peptides, powdered egg yolk, potassium caseinate, unphosphorylated peptides, casein peptides, mixed caseinate, soya isolate and mixtures thereof.

9. Composition according to any one of claims 1 to 8, characterized in that the said lipids belong to the group comprising butter oil, peanut oil, medium chain triglycerides, grape seed oil, soya oil, evening primrose oil and mixtures thereof.

10. Composition according to one of claims 1 to 9, characterized in that the said lipids consist of a mixture of at least one oil of animal origin, at least one oil of vegetable origin and glycerol stearate.

11. Composition according to one of claims 1 and 6 to 10, characterized in that it constitutes the daily food ration of a human being and in that it comprises :
- between 75 g and 500 g of glucides,
- between 20 g and 185 g of lipids,
- between 20 g and 225 g of proteins,
- vitamins, minerals and electrolytes in sufficient quantities to satisfy the daily nutritional needs of a human being.

12. Composition according to one of claims 1 to 10, characterized in that it is the daily food ration of a human being and in that it comprises :
- less than 50 g and preferably between 1 and 10 g of the said inhibitor of the intracellular synthesis of polyamines,
- between 75 g and 500 g of glucides,
- between 20 g and 185 g of lipids,
- between 20 g and 225 g of proteins,
- vitamins, minerals and electrolytes in sufficient quantities to satisfy the daily nutritional needs of a human being.

13. Composition according to one of claims 1 and 6 to 10, characterized in that it is a sub-multiple of the daily food ration of a human being and comprises :
- between 75/X g and 500/X g of glucides,
- between 20/X g and 185/X g of lipids,
- between 20/X g and 225/X g of proteins,
- vitamins, minerals and electrolytes in sufficient quantities to satisfy the daily nutritional needs of a human being,
X being an integer between 2 and 8, and corresponding to the number of rations which must be ingested by the patient to satisfy his or her daily nutritional needs.

14. Composition according to one of claims 1 to 10, characterized in that it is a sub-multiple of a daily food ration of a human being and in that it comprises :
- less than 50/X g and preferably between 1/X and 10/X g of the said inhibitor of the intracellular synthesis of polyamines,
- between 75/X g and 500/X g of glucides,
- between 20/X g and 185/X g of lipids,
- between 20/X g and 225/X g of proteins,
- vitamins, minerals and electrolytes in sufficient quantities to satisfy the daily nutritional needs of a human being,
X being an integer between 2 and 8, and corresponding to the number of rations which must be ingested by the patient to satisfy his or her daily nutritional needs.

15. Composition according to one of claims 1 to 14, characterized in that it is in a dry form to be dissolved extemporaneously in a neutral vehicle.

16. Composition according to one of claims 1 to 14, characterized in that it includes a neutral vehicle making it ready for use.

17. Agent with two components A and B, component A consisting of a composition that can be ingested by man according to one of claims 1, 6 to 11, 13, 15 or 16, and compound B consisting of an inhibitor of the intracellular synthesis of polyamines, components A and B being used as combined products for use simultaneously, separately or with a time lag.

18. Agent according to claim 17, characterized in that the said inhibitor is α-methylornithine.

19. Composition or agent according to one of claims 1 to 18 for a use belonging to the group including foods, food supplements and nutritional products.

20. Composition or agent according to any one of claims 1 to 18, which can be used as a drug.

21. Composition or agent according to either of claims 19 or 20, which can be used as a therapeutic nutritional product.

22. Composition according to any one of claims 19 to 21, which can be used as an anti-cancer agent.

23. Composition or agent according to claim 22, which can be used for the treatment of cancer of the prostate.

## Patentansprüche

1. Vom Menschen einnehmbare Zusammensetzung,
dadurch gekennzeichnet, daß sie aus einem polyaminarmen Nährstoffgemisch besteht, das weniger als 50 Pikomol/g Putrescin, Spermidin, Spermin und Kadaverin in Prozent Trockengewicht im Verhältnis zum Gesamttrockengewicht enthält: 10 % bis 35 % Lipide, 8 % bis 30 % Proteine, 35 % bis 80 % Kohlehydrate und bis zu 10 % eines Gemisches, welches aus Vitaminen, Mineralstoffen und Elektrolyten besteht.

2. Zusammensetzung gemäß Anspruch 1,
dadurch gekennzeichnet, daß sie mit mindestens einem Hemmstoff der Polyamin-Innenzellsynthese angereichert ist, im Verhältnis von höchstens 15 % bezüglich des Gesamttrockengewichtes der Zusammensetzung.

3. Zusammensetzung gemäß Anspruch 2,
dadurch gekennzeichnet, daß sie mit dem bereits erwähnten Hemmstoff im Verhältnis von 0,2 % bis 0,3 % im Gewicht bezüglich des Gesamttrockengewichtes der Zusammensetzung angereichert ist.

4. Zusammensetzung gemäß Anspruch 2 oder 3,
dadurch gekennzeichnet, daß es sich bei dem Hemmstoff um einen kompetitiven Inhibitor der Ornithyn Decarboxylase handelt.

5. Zusammensetzung gemäß Anspruch 4,
dadurch gekennzeichnet, daß es sich beim kompetitiven Inhibitor um α-Methylornithin handelt.

6. Zusammensetzung gemäß einem der Ansprüche 1 bis 5,
dadurch gekennzeichnet, daß sie mit Vitaminen angereichert ist.

7. Zusammensetzung gemäß einem der Ansprüche 1 bis 6,
dadurch gekennzeichnet, daß die Kohlehydrate der Gruppe angehören, welche Glucosepolymere, Maltodextrine, Saccharosen, geänderte Stärken, Monohydratglucose, Glucosesyrup, Glycerinmonostearat und deren Mischungen enthält.

8. Zusammensetzung gemäß einem der Ansprüche 1 bis 7,
dadurch gekennzeichnet, daß die Proteine der Gruppe angehören, welche lösliche Milchproteine, Sojaproteine, Serumpeptide, Eigelbpulver, Kaliumkaseinat, nicht phosphorylierte Peptide, Kaseinpeptide, gemischtes Kaseinat, Sojaisolat und deren Mischungen umfaßt.

9. Zusammensetzung gemäß einem der Ansprüche 1 bis 8,
dadurch gekennzeichnet, daß die Lipide der Gruppe angehören, welche Butteröl, Erdnußöl, Triglyzeride mit mittleren Ketten, Traubenkernöl, Sojaöl, Oenotheraöl und deren Mischungen umfaßt.

10. Zusammensetzung gemäß einem der Ansprüche 1 bis 9,
dadurch gekennzeichnet, daß die Lipide aus einer Mischung von mindestens einem tierischen Öl, mindestens einem pflanzlichen Öl und Glyzerinstearat bestehen.

11. Zusammensetzung gemäß einem der Ansprüche 1 und 6 bis 10,
dadurch gekennzeichnet, daß sie die tägliche Nahrungsration eines Menschen darstellt, wobei sie folgendes enthält:
- 75 g bis 500 g Kohlehydrate,
- 20 g bis 185 g Lipide,
- 20 g bis 225 g Proteine,
- Vitamine, Mineralstoffe und Elektrolyte in ausreichenden Mengen, um den täglichen Nährstoffbedarf eines Menschen zu befriedigen.

12. Zusammensetzung gemäß einem der Ansprüche 1 bis 10,
dadurch gekennzeichnet, daß sie die tägliche Nahrungsration eines Menschen darstellt, wobei sie folgendes enthält:
- weniger als 50 g, bevorzugterweise 1 bis 10 g des Hemmstoffes der Polyamin-Innenzellsynthese,
- 75 g bis 500 g Kohlehydrate,
- 20 g bis 185 g Lipide,
- 20 g bis 225 g Proteine,
- Vitamine, Mineralstoffe und Elektrolyte in ausreichenden Mengen, um den täglichen Nährstoffbedarf eines Menschen zu befriedigen.

13. Zusammensetzung gemäß einem der Ansprüche 1 und 6 bis 10,
dadurch gekennzeichnet, daß sie die tägliche Nahrungsration eines Menschen darstellt, wobei sie folgendes enthält:
- 75/X g bis 500/X g Kohlehydrate,
- 20/X g bis 185/X g Lipide,
- 20/X g bis 225/X g Proteine,
- Vitamine, Mineralstoffe und Elektrolyte in ausreichenden Mengen, um partiell den täglichen Nährstoffbedarf eines Menschen zu befriedigen
und
wobei X zwischen 2 und 8 liegt und der Zahl der Rationen entspricht, die vom Patienten einzunehmen sind, um seinen täglichen Nährstoffbedarf zu befriedigen.

14. Zusammensetzung gemäß einem der Ansprüche 1 bis 10,
dadurch gekennzeichnet, daß sie ein ganzer Bruchteil einer täglichen Nährstoffration eines Menschen ist und dadurch, daß sie folgendes umfaßt:
- weniger als 50/X g, bevorzugterweise 1/X bis 10/X g des Hemmstoffes der Polyamin-Innenzellsynthese,
- 75/X g bis 500/X g Kohlehydrate,
- 20/X g bis 185/X g Lipide,
- 20/X g bis 225/X g Proteine,
- Vitamine, Mineralstoffe und Elektrolyte in ausreichenden Mengen, um partiell den täglichen Nährstoffbedarf eines Menschen zu befriedigen.
und
wobei X eine ganze Zahl ist und zwischen 2 und 8 liegt und der Zahl der Rationen entspricht, die vom Patienten einzunehmen sind, um seinen täglichen Nährstoffbedarf zu befriedigen.

15. Zusammensetzung gemäß einem der Ansprüche 1 bis 14,
dadurch gekennzeichnet, daß sie in trockener Form vorliegt, die nach Bedarf in einem neutralen Trägerstoff gelöst wird.

16. Zusammensetzung gemäß einem der Ansprüche 1 bis 14,
dadurch gekennzeichnet, daß sie einen neutralen Trägerstoff umfaßt, wodurch sie gebrauchsfertig ist.

17. Mittel mit zwei Komponenten A und B, wobei die Komponente A aus einer Zusammensetzung besteht, die vom Menschen eingenommen werden kann, gemäß einem der Ansprüche 1, 6 bis 11, 13, 15 oder 16 und wobei die Komponente B aus einem Hemmstoff der Polyamin-Innenzellsynthese besteht, wobei die Komponenten A und B als Kombinationsprodukte zur gleichzeitigen Anwendung eingesetzt werden, die zeitlich getrennt oder verschoben ist.

18. Mittel gemäß Anspruch 17,
dadurch gekennzeichnet, daß es sich beim Hemmstoff um α-Methylornithin handelt.

19. Zusammensetzung oder Mittel gemäß einem der Ansprüche 1 bis 18, für eine Anwendung, die der Gruppe angehört, die Nährmittel, Nährmittelzusätze und Nährstoffe umfaßt.

20. Zusammensetzung oder Mittel gemäß einem der Ansprüche 1 bis 18, die/das als Medikament einsetzbar ist.

21. Zusammensetzung oder Mittel gemäß einem der Ansprüche 19 oder 20, die/das als therapeutischer Nährstoff einsetzbar ist.

22. Zusammensetzung oder Mittel gemäß einem der Ansprüche 19 bis 21, die/das als krebshemmender Stoff einsetzbar ist.

23. Zusammensetzung oder Mittel gemäß Anspruch 22, die/das zur Behandlung von Prostatakrebs einsetzbar ist.
